# EUROPEAN PATENT SPECIFICATION

(11) **EP 2 850 436 B1**
(45) Date of publication and mention of the grant of the patent: **05.07.2017**
(21) Application number: 13731914.1
(22) Date of filing: 13.05.2013
(51) Int. Cl.: G01N 33/68

(54) **AN IN VITRO METHOD FOR DIAGNOSING OF ENDOMETRIOSIS**
IN-VITRO-VERFAHREN ZUR DIAGNOSE VON ENDOMETRIOSE
PROCÉDÉ IN VITRO POUR LE DIAGNOSTIC DE L'ENDOMÉTRIOSE

(30) Priority: 14.05.2012 IT RM20120214
(43) Date of publication of application: 25.03.2015
(73) Proprietor: Signorile, Pietro Giulio, 00155 Roma (IT); Baldi, Alfonso, 80128 Napoli (IT)
(72) Inventor: Signorile, Pietro Giulio, 00155 Roma (IT); Baldi, Alfonso, 80128 Napoli (IT)
(74) Representative: Di Giovine, Paolo
(86) International application number: PCT/IB2013/053875
(87) International publication number: WO 2013/171655

(56) References cited:
- WO-A1-00/55350
- WO-A1-2011/020839
- WO-A2-01/32920
- WO-A2-2005/008251
- WO-A2-2011/161427
- WO-A2-2012/088290
- US-A1- 2002 119 143
- US-A1- 2009 220 994
- ABDUL-RAHMAN P S ET AL: "Expression of High Abundance Proteins in Sera of Patients with Endometrial and Cervical Cancer: Analysis Using 2-DE with Silver Staining and Lectin Detection Methods", ELECTROPHORESIS,, vol. 28, no. 12, 12 June 2007 (2007-06-12) , pages 1989-1996, XP008109667, DOI: 10.1002/ELPS.200600629

### SEQUENCE LISTING

<110> signorile/baldi, pietrogiulio/alfonso
<120> An in vitro method for diagnosing endometriosis
<130> BW708R
<160> 5
<170> BiSSAP 1.0
<210> 1
   <211> 317
   <212> PRT
   <213> Homo sapiens
<220>
   <221> SOURCE
   <222> 1..317
   <223> /mol_type="protein" /organism="Homo sapiens"
<400> 1
   Met Lys Val Leu Trp Ala Ala Leu Leu Val Thr Phe Leu Ala Gly Cys
   1               5                   10                  15
   Gln Ala Lys Val Lys Gln Ala Val Glu Thr Glu Pro Glu Pro Glu Leu
               20                  25                  30
   Arg Gln Gln Thr Glu Trp Gln Ser Gly Gln Arg Trp Glu Leu Ala Leu
           35                  40                  45
   Gly Arg Phe Trp Asp Tyr Leu Arg Trp Val Gln Thr Leu Ser Glu Gln
       50                  55                  60
   Val Gln Glu Glu Leu Leu Ser Ser Gln Val Thr Gln Glu Leu Arg Ala
   65                  70                  75                  80
   Leu Met Asp Glu Thr Met Lys Glu Leu Lys Ala Tyr Lys Ser Glu Leu
                   85                  90                  95
   Glu Glu Gln Leu Thr Pro Val Ala Glu Glu Thr Arg Ala Arg Leu Ser
               100                 105                 110
   Lys Glu Leu Gln Ala Ala Gln Ala Arg Leu Gly Ala Asp Met Glu Asp
           115                 120                 125
   Val Cys Gly Arg Leu Val Gln Tyr Arg Gly Glu Val Gln Ala Met Leu
       130                 135                 140
   Gly Gln Ser Thr Glu Glu Leu Arg Val Arg Leu Ala Ser His Leu Arg
   145                 150                 155                 160
   Lys Leu Arg Lys Arg Leu Leu Arg Asp Ala Asp Asp Leu Gln Lys Arg
                   165                 170                 175
   Leu Ala Val Tyr Gln Ala Gly Ala Arg Glu Gly Ala Glu Arg Gly Leu
               180                 185                 190
   Ser Ala Ile Arg Glu Arg Leu Gly Pro Leu Val Glu Gln Gly Arg Val
           195                 200                 205
   Arg Ala Ala Thr Val Gly Ser Leu Ala Gly Gln Pro Leu Gln Glu Arg
       210                 215                 220
   Ala Gln Ala Trp Gly Glu Arg Leu Arg Ala Arg Met Glu Glu Met Gly
   225                 230                 235                 240
   Ser Arg Thr Arg Asp Arg Leu Asp Glu Val Lys Glu Gln Val Ala Glu
                   245                 250                 255
   Val Arg Ala Lys Leu Glu Glu Gln Ala Gln Gln Ile Arg Leu Gln Ala
               260                 265                 270
   Glu Ala Phe Gln Ala Arg Leu Lys Ser Trp Phe Glu Pro Leu Val Glu
           275                 280                 285
   Asp Met Gln Arg Gln Trp Ala Gly Leu Val Glu Lys Val Gln Ala Ala
       290                 295                 300
   Val Gly Thr Ser Ala Ala Pro Val Pro Ser Asp Asn His
             305                 310                 315
<210> 2
   <211> 432
   <212> PRT
   <213> Homo sapiens
<220>
   <221> SOURCE
   <222> 1..432
   <223> /mol_type="protein" /organism="Homo sapiens"
<400> 2
<210> 3
   <211> 585
   <212> PRT
   <213> Homo sapiens
<220>
   <221> SOURCE
   <222> 1..585
   <223> /mol_type="protein" /organism="Homo sapiens"
<400> 3
   Asp Ala His Lys Ser Glu Val Ala His Arg Phe Lys Asp Leu Gly Glu
   1               5                   10                  15
   Glu Asn Phe Lys Ala Leu Val Leu Ile Ala Phe Ala Gln Tyr Leu Gln
               20                  25                  30
   Gln Cys Pro Phe Glu Asp His Val Lys Leu Val Asn Glu Val Thr Glu
           35                  40                  45
   Phe Ala Lys Thr Cys Val Ala Asp Glu Ser Ala Glu Asn Cys Asp Lys
       50                  55                  60
   Ser Leu His Thr Leu Phe Gly Asp Lys Leu Cys Thr Val Ala Thr Leu
   65                  70                  75                  80
   Arg Glu Thr Tyr Gly Glu Met Ala Asp Cys Cys Ala Lys Gln Glu Pro
                   85                  90                  95
   Glu Arg Asn Glu Cys Phe Leu Gln His Lys Asp Asp Asn Pro Asn Leu
               100                 105                 110
   Pro Arg Leu Val Arg Pro Glu Val Asp Val Met Cys Thr Ala Phe His
           115                 120                 125
   Asp Asn Glu Glu Thr Phe Leu Lys Lys Tyr Leu Tyr Glu Ile Ala Arg
       130                 135                 140
   Arg His Pro Tyr Phe Tyr Ala Pro Glu Leu Leu Phe Phe Ala Lys Arg
   145                 150                 155                 160
   Tyr Lys Ala Ala Phe Thr Glu Cys Cys Gln Ala Ala Asp Lys Ala Ala
                   165                 170                 175
   Cys Leu Leu Pro Lys Leu Asp Glu Leu Arg Asp Glu Gly Lys Ala Ser
               180                 185                 190
   Ser Ala Lys Gln Arg Leu Xaa Cys Ala Ser Leu Gln Lys Phe Gly Glu
           195                 200                 205
   Arg Ala Phe Lys Ala Trp Ala Val Ala Arg Leu Ser Gln Arg Phe Pro
       210                 215                 220
   Lys Ala Glu Phe Ala Glu Val Ser Lys Leu Val Thr Asp Leu Thr Lys
   225                 230                 235                 240
   Val His Thr Glu Cys Cys His Gly Asp Leu Leu Glu Cys Ala Asp Asp
                   245                 250                 255
   Arg Ala Asp Leu Ala Lys Tyr Ile Cys Glu Asn Gln Asp Ser Ile Ser
               260                 265                 270
   Ser Lys Leu Lys Glu Cys Cys Glu Lys Pro Leu Leu Glu Lys Ser His
           275                 280                 285
   Cys Ile Ala Glu Val Glu Asn Asp Glu Met Pro Ala Asp Leu Pro Ser
       290                 295                 300
   Leu Ala Ala Asp Phe Val Glu Ser Lys Asp Val Cys Lys Asn Tyr Ala
   305                 310                 315                 320
   Glu Ala Lys Asp Val Phe Leu Gly Met Phe Leu Tyr Glu Tyr Ala Arg
                   325                 330                 335
   Arg His Pro Asp Tyr Ser Val Val Leu Leu Leu Arg Leu Ala Lys Thr
               340                 345                 350
   Tyr Glu Thr Thr Leu Glu Lys Cys Cys Ala Ala Ala Asp Pro His Glu
           355                 360                 365
   Cys Tyr Ala Lys Val Phe Asp Glu Phe Lys Pro Leu Val Glu Glu Pro
       370                 375                 380
   Gln Asn Leu Ile Lys Gln Asn Cys Glu Leu Phe Glu Gln Leu Gly Glu
   385                 390                 395                 400
   Tyr Lys Phe Gln Asn Ala Leu Leu Val Arg Tyr Thr Lys Lys Val Pro
                   405                 410                 415
   Gln Val Ser Thr Pro Thr Leu Val Glu Val Ser Arg Asn Leu Gly Lys
               420                 425                 430
   Val Gly Ser Lys Cys Cys Lys His Pro Glu Ala Lys Arg Met Pro Cys
           435                 440                 445
   Ala Glu Asp Tyr Leu Ser Val Val Leu Asn Gln Leu Cys Val Leu His
       450                 455                 460
   Glu Lys Thr Pro Val Ser Asp Arg Val Thr Lys Cys Cys Thr Glu Ser
   465                 470                 475                 480
   Leu Val Asn Arg Arg Pro Cys Phe Ser Ala Leu Glu Val Asp Glu Thr
                   485                 490                 495
   Tyr Val Pro Lys Glu Phe Asn Ala Glu Thr Phe Thr Phe His Ala Asp
               500                 505                 510
   Ile Cys Thr Leu Ser Glu Lys Glu Arg Gln Ile Lys Lys Gln Thr Ala
           515                 520                 525
   Leu Val Glu Leu Val Lys His Lys Pro Lys Ala Thr Lys Glu Gln Leu
       530                 535                 540
   Lys Ala Val Met Asp Asp Phe Ala Ala Phe Val Glu Lys Cys Cys Lys
   545                 550                 555                 560
   Ala Asp Asp Lys Glu Thr Cys Phe Ala Glu Glu Gly Lys Lys Leu Val
                   565                 570                 575
   Ala Ala Ser Gln Ala Ala Leu Gly Leu
               580                 585
<210> 4
   <211> 1663
   <212> PRT
   <213> Homo sapiens
<220>
   <221> SOURCE
   <222> 1..1663
   <223> /mol_type="protein" /organism="Homo sapiens"
<400> 4
<210> 5
   <211> 763
   <212> PRT
   <213> Homo sapiens
<220>
   <221> SOURCE
   <222> 1..763
   <223> /mol_type="protein" /organism="Homo sapiens"
<400> 5
   Met Arg Leu Leu Trp Gly Leu Ile Trp Ala Ser Ser Phe Phe Thr Leu
   1               5                   10                  15
   Ser Leu Gln Lys Pro Arg Leu Leu Leu Phe Ser Pro Ser Val Val His
               20                  25                  30
   Leu Gly Val Pro Leu Ser Val Gly Val Gln Leu Gln Asp Val Pro Arg
           35                  40                  45
   Gly Gln Val Val Lys Gly Ser Val Phe Leu Arg Asn Pro Ser Arg Asn
       50                  55                  60
   Asn Val Pro Cys Ser Pro Lys Val Asp Phe Thr Leu Ser Ser Glu Arg
   65                  70                  75                  80
   Asp Phe Ala Leu Leu Ser Leu Gln Val Pro Leu Lys Asp Ala Lys Ser
                   85                  90                  95
   Cys Gly Leu His Gln Leu Leu Arg Gly Pro Glu Val Gln Leu Val Ala
               100                 105                 110
   His Ser Pro Trp Leu Lys Asp Ser Leu Ser Arg Thr Thr Asn Ile Gln
           115                 120                 125
   Gly Ile Asn Leu Leu Phe Ser Ser Arg Arg Gly His Leu Phe Leu Gln
       130                 135                 140
   Thr Asp Gln Pro Ile Tyr Asn Pro Gly Gln Arg Val Arg Tyr Arg Val
   145                 150                 155                 160
   Phe Ala Leu Asp Gln Lys Met Arg Pro Ser Thr Asp Thr Ile Thr Val
                   165                 170                 175
   Met Val Glu Asn Ser His Gly Leu Arg Val Arg Lys Lys Glu Val Tyr
               180                 185                 190
   Met Pro Ser Ser Ile Phe Gln Asp Asp Phe Val Ile Pro Asp Ile Ser
           195                 200                 205
   Glu Pro Gly Thr Trp Lys Ile Ser Ala Arg Phe Ser Asp Gly Leu Glu
       210                 215                 220
   Ser Asn Ser Ser Thr Gln Phe Glu Val Lys Lys Tyr Val Leu Pro Asn
   225                 230                 235                 240
   Phe Glu Val Lys Ile Thr Pro Gly Lys Pro Tyr Ile Leu Thr Val Pro
                   245                 250                 255
   Gly His Leu Asp Glu Met Gln Leu Asp Ile Gln Ala Arg Tyr Ile Tyr
               260                 265                 270
   Gly Lys Pro Val Gln Gly Val Ala Tyr Val Arg Phe Gly Leu Leu Asp
           275                 280                 285
   Glu Asp Gly Lys Lys Thr Phe Phe Arg Gly Leu Glu Ser Gln Thr Lys
       290                 295                 300
   Leu Val Asn Gly Gln Ser His Ile Ser Leu Ser Lys Ala Glu Phe Gln
   305                 310                 315                 320
   Asp Ala Leu Glu Lys Leu Asn Met Gly Ile Thr Asp Leu Gln Gly Leu
                   325                 330                 335
   Arg Leu Tyr Val Ala Ala Ala Ile Ile Glu Ser Pro Gly Gly Glu Met
               340                 345                 350
   Glu Glu Ala Glu Leu Thr Ser Trp Tyr Phe Val Ser Ser Pro Phe Ser
           355                 360                 365
   Leu Asp Leu Ser Lys Thr Lys Arg His Leu Val Pro Gly Ala Pro Phe
       370                 375                 380
   Leu Leu Gln Ala Leu Val Arg Glu Met Ser Gly Ser Pro Ala Ser Gly
   385                 390                 395                 400
   Ile Pro Val Lys Val Ser Ala Thr Val Ser Ser Pro Gly Ser Val Pro
                   405                 410                 415
   Glu Val Gln Asp Ile Gln Gln Asn Thr Asp Gly Ser Gly Gln Val Ser
               420                 425                 430
   Ile Pro Ile Ile Ile Pro Gln Thr Ile Ser Glu Leu Gln Leu Ser Val
           435                 440                 445
   Ser Ala Gly Ser Pro His Pro Ala Ile Ala Arg Leu Thr Val Ala Ala
       450                 455                 460
   Pro Pro Ser Gly Gly Pro Gly Phe Leu Ser Ile Glu Arg Pro Asp Ser
   465                 470                 475                 480
   Arg Pro Pro Arg Val Gly Asp Thr Leu Asn Leu Asn Leu Arg Ala Val
                   485                 490                 495
   Gly Ser Gly Ala Thr Phe Ser His Tyr Tyr Tyr Met Ile Leu Ser Arg
               500                 505                 510
   Gly Gln Ile Val Phe Met Asn Arg Glu Pro Lys Arg Thr Leu Thr Ser
           515                 520                 525
   Val Ser Val Phe Val Asp His His Leu Ala Pro Ser Phe Tyr Phe Val
       530                 535                 540
   Ala Phe Tyr Tyr His Gly Asp His Pro Val Ala Asn Ser Leu Arg Val
   545                 550                 555                 560
   Asp Val Gln Ala Gly Ala Cys Glu Gly Lys Leu Glu Leu Ser Val Asp
                   565                 570                 575
   Gly Ala Lys Gln Tyr Arg Asn Gly Glu Ser Val Lys Leu His Leu Glu
               580                 585                 590
   Thr Asp Ser Leu Ala Leu Val Ala Leu Gly Ala Leu Asp Thr Ala Leu
           595                 600                 605
   Tyr Ala Ala Gly Ser Lys Ser His Lys Pro Leu Asn Met Gly Lys Val
       610                 615                 620
   Phe Glu Ala Met Asn Ser Tyr Asp Leu Gly Cys Gly Pro Gly Gly Gly
   625                 630                 635                 640
   Asp Ser Ala Leu Gln Val Phe Gln Ala Ala Gly Leu Ala Phe Ser Asp
                   645                 650                 655
   Gly Asp Gln Trp Thr Leu Ser Arg Lys Arg Leu Ser Cys Pro Lys Glu
               660                 665                 670
   Lys Thr Thr Arg Lys Lys Arg Asn Val Asn Phe Gln Lys Ala Ile Asn
           675                 680                 685
   Glu Lys Leu Gly Gln Tyr Ala Ser Pro Thr Ala Lys Arg Cys Cys Gln
       690                 695                 700
   Asp Gly Val Thr Arg Leu Pro Met Met Arg Ser Cys Glu Gln Arg Ala
   705                 710                 715                 720
   Ala Arg Val Gln Gln Pro Asp Cys Arg Glu Pro Phe Leu Ser Cys Cys
                   725                 730                 735
   Gln Phe Ala Glu Ser Leu Arg Lys Lys Ser Arg Asp Lys Gly Gln Glu
               740                 745                 750
   Gly Leu Gly Ser Pro Ser Ala Arg Ser Pro Ala
           755                 760

## Description

The present description relates to an *in vitro* method for the diagnosis of endometriosis, an *in vitro* method for the monitoring of a therapy against endometriosis and the use of a kit for the diagnosis of endometriosis and/or for the monitoring of a therapy against endometriosis in a subject.

### STATE OF THE PRIOR ART

Endometriosis is a pathological condition characterized by the presence of endometrial tissue outside the uterus, and is associated to pelvic pain and infertility (Giudice LC, and Kao LC: Endometriosis. The Lancet, 364: 1789-1799, 2004). Its incidence is estimated at about 10% of the female population of reproductive age (Houston DE: Evidence for the risk of pelvic endometriosis by age, race, and socioeconomic status. Epidemiol Rev, 6: 167-191, 1984.). The symptoms are, in general, not very specific, and often erroneously attributed to other pathologies causing chronic pains; therefore, a diagnosis of endometriosis is hardly performed at the earliest stages of the disease. Moreover, a diagnosis of certainty can be carried out only through an invasive approach, consisting in a laparoscopic inspection and histological analysis of suspect lesions. Therefore, the diagnosis of endometriosis is always posed with significant delay: current estimates indicate a time interval of 8-12 years between symptoms appearance and the diagnosis of endometriosis (Hadfield R, Mardon H, Barlow D, Kennedy S. Delay in the diagnosis of endometriosis: a survey of women from the USA and the UK. Hum Reprod 1996, 11: 878-880.). At present, there is no possibility of carrying out an early diagnosis of endometriosis through the use of less invasive methods. A recent review of the literature on the topic highlighted how, despite the great number of scientific publications on the topic, to date there is no biomarker or group of biomarkers that proved clinically effective to pose the diagnosis of endometriosis by use of non-invasive methodologies, like, for instance, a sample of peripheral blood (May KE, Conduit-Hulbert SA, Villar J, Kirtkley S, Kennedy SH, Becker CM: Peripheral biomarkers of endometriosis: a systematic review. Hum Reprod 2010, 16: 651-674). A very recent work by Fassbender et al. (Fassbender A, Waelkens E, Verbeeck N, Kyama CM, Bokor A, Vodolazkaia A, Van de Plas R, Meuleman C, Peeraer K, Tomassetti C, Gevaert O, Ojeda F, De Moor B, D'Hooghe T: Proteomics analysis of plasma for early diagnosis of endometriosis. Obstet Gynecol 2012, 119: 276-285) has singled out, through proteomics analyses based on the MALDI-TOFF methodology, a group of polypeptide peaks whose expression in blood seems to correlate with the presence or absence of endometriosis. However, the authors, though observing a variation in peak patterns of about 10 different peptides/proteins in various stages of endometriosis (from minimal to severe stage) do not identify the origin of such proteins, which therefore remain unknown and, as such, not useful to define a group of biomarkers whose variation of expression may be considered advantageous for diagnostic purposes.
WO2011020839 discloses a method of predicting response to thalidomide in a multiple myeloma patient.
US2009220994 discloses methods for diagnosis of chronic prostatitis/chronic pain pelvic syndrome.
WO0132920 discloses the discovery of genes and their products that are associated with the disease endometriosis

WO2005008251 discloses marker polypeptides associated with endometriosisHence, in the state of the known art the need of singling out non-invasive diagnostic instruments allowing an accurate, and possibly early, diagnosis of endometriosis in order to define a therapeutic strategy already in the early stages of disease development is highly felt.

### SUMMARY OF THE INVENTION

The present description relates to an *in vitro* method and the use of a kit for the diagnosis of endometriosis.

The present invention is based on the discovery that the expression of a group of proteins, detailed in the next section, varies in a statistically significant manner in patients suffering from endometriosis with respect to the population of healthy controls.

In particular, the observation of the Inventors about the quantitative difference of the expression of the proteins at issue in subjects also in the earliest stages of the pathology with respect to healthy control patients, enabled to define for the first time a method for evaluating the presence of said pathology. One of the advantages of the present invention therefore consists in the possibility of reaching a diagnosis significantly ahead of the onset of symptoms typical of this pathology.

Therefore, the observation of the variation of the concentration of such proteins can be advantageously utilized for defining an *in vitro* method for the diagnosis of endometriosis in female subjects. Such variation of expression is observed particularly on blood samples of subjects with endometriosis. Therefore, even more advantageously, said method, in an embodiment thereof, is carried out in an absolutely noninvasive manner thanks to the fact that the determining of the concentration of the proteins of interest can be performed on a blood sample. In particular, taking into account that for a firm diagnosis of endometriosis currently methods are available which envisage invasive surgical practices aimed at obtaining a sample of endometrial tissue, it can be stated that, with respect to the state of the known art, in an embodiment, the diagnostic method described herein attains a considerable technical advancement consisting in a remarkable increase of compliance by subjects under examination.
Therefore, a first object of the present application is:
- an *in vitro* method for the diagnosis of endometriosis in a subject, comprising the following steps:
   a) determining the concentration of at least one protein comprising or consisting in a sequence selected from the group of: SEQ ID NO 1, SEQ ID NO 2, SEQ ID NO 3, SEQ ID NO 4, SEQ ID NO 5 and SEQ ID NO 6, or mutants and/or post-translational variants thereof, in a biological sample of said subject and in a control sample;
   b) comparing said concentrations in said biological sample and said control sample
wherein an increase of the concentration of said at least one protein comprising or consisting in a sequence selected from the group of SEQ ID NO 2, SEQ ID NO 3, SEQ ID NO 6 and/or a decrease of the concentration of said at least one protein comprising or consisting in a sequence selected from the group of SEQ ID NO 1, SEQ ID NO 4, SEQ ID NO 5 with respect to the same protein in said control is indicative of endometriosis.

A second object of the present description is:
- an *in vitro* method for the monitoring of a therapy against endometriosis in a subject under said therapy, comprising the following steps:
   a) determining the concentration of at least one protein comprising or consisting in a sequence selected from the group of: SEQ ID NO 1, SEQ ID NO 2, SEQ ID NO 3, SEQ ID NO 4, SEQ ID NO 5 and SEQ ID NO 6, or mutants and/or post-translational variants thereof, in a first and in a second biological sample, obtained at different times, of said subject,
   b) comparing said concentration obtained for said first and second sample.

Object of the present description is also the use of a kit for the diagnosis of endometriosis and/or the monitoring of a therapy against endometriosis in a subject, comprising:
- at least one aliquot of one or more reagents necessary to the determining of the concentration of at least one protein comprising or consisting in a sequence selected from the group of: SEQ ID NO 1, SEQ ID NO 2, SEQ ID NO 3, SEQ ID NO 4, SEQ ID NO 5 and SEQ ID NO 6 or mutants and/or post-translational variants thereof in a biological sample of said subject.

Further advantages, as well as the features and the operation steps of the present invention will be made apparent in the following detailed description of some preferred embodiments thereof, given merely by way of example and not for limitative purposes.

### DETAILED DESCRIPTION OF THE FIGURES

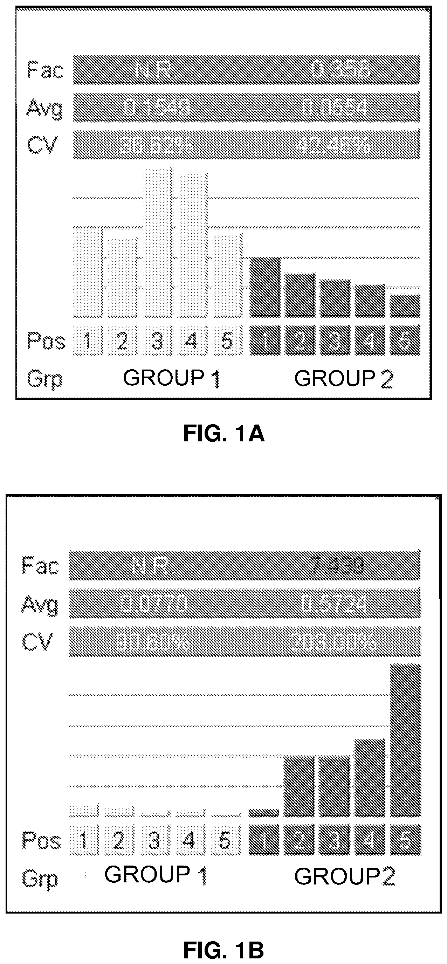

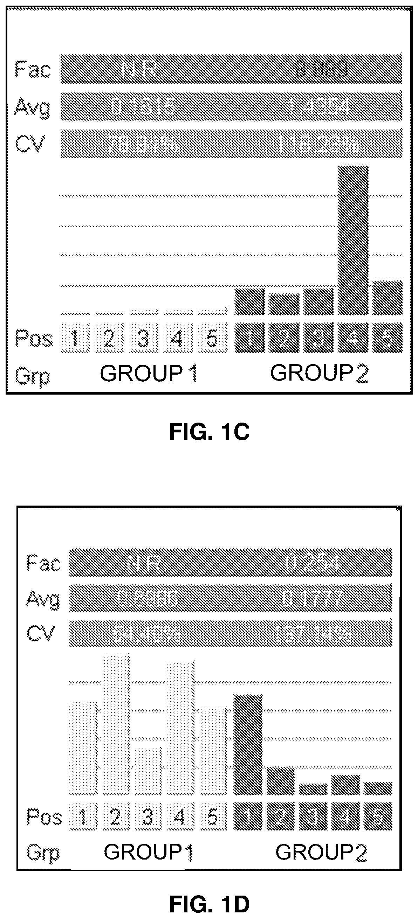

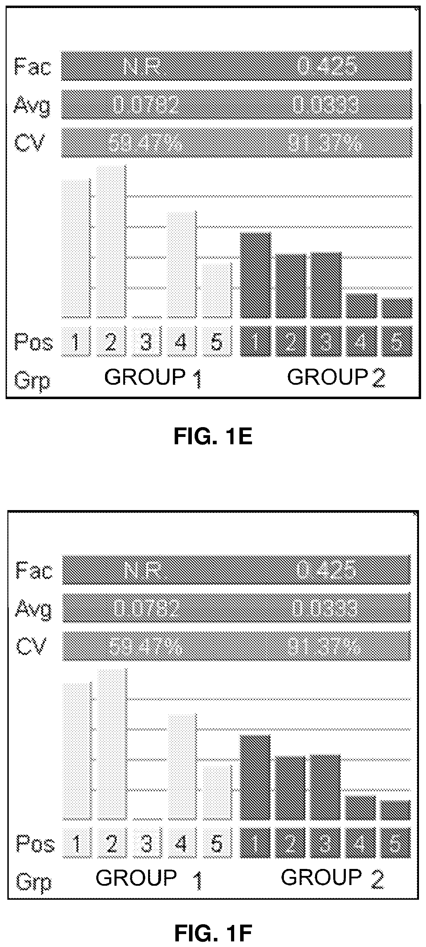

Figures 1A, 1B, 1C, 1D. 1E and 1F show histograms related to the different expression respectively of proteins of SEQ ID NO: 1, SEQ ID NO: 2, SEQ ID NO: 3, SEQ ID NO: 4, SEQ ID NO: 5 and SEQ ID NO: 6 in serum samples of patients with endometriosis (group 2) with respect to the serum samples of a control population (group 1). CV= variation coefficient, AVG= average value, Fac= regulation factor.

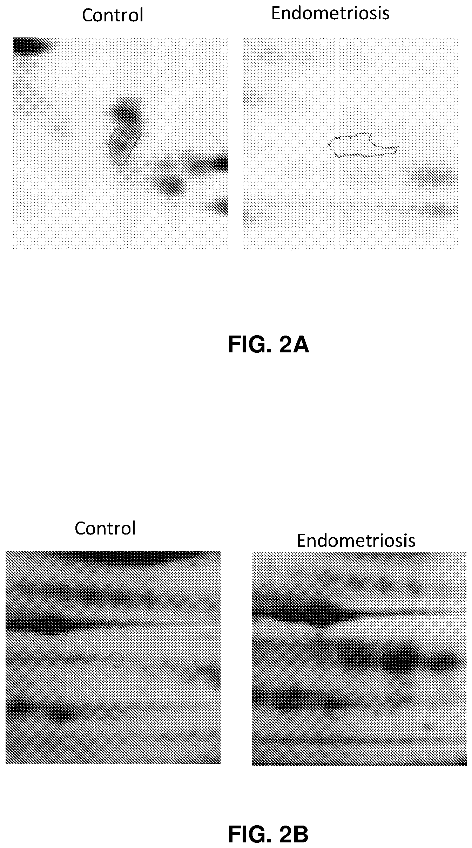

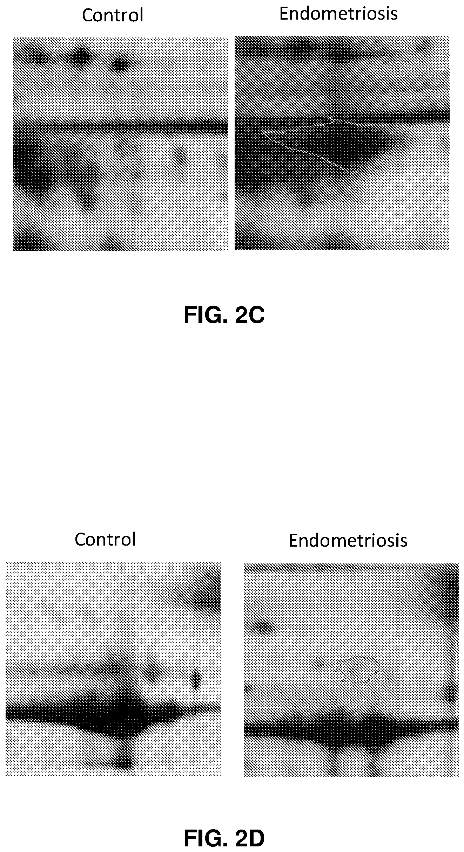

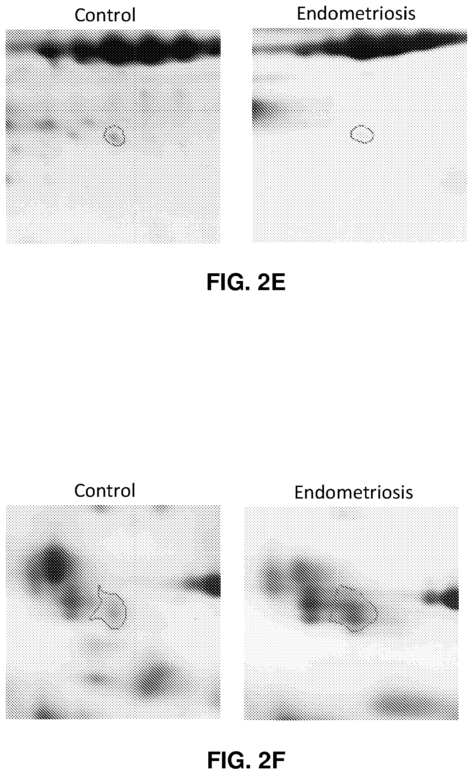

Figures 2A-F show a two-dimensional gel in which are visible the various expression levels of proteins of respectively SEQ ID NO: 1, SEQ ID NO: 2, SEQ ID NO: 3, SEQ ID NO: 4, SEQ ID NO: 5 and SEQ ID NO: 6 in control samples and in samples of patients with endometriosis.

### SEQUENCE DESCRIPTION

**SEQ ID NO 1** Protein corresponding to human apolipoprotein E mutant E3K (Accession number in GenBank: GI 1506383A; 317 amino acids):
   mkvlwaallv tflagcqakv kqavetepep elrqqtewqs gqrwelalgr fwdylrwvqt Iseqvqeell ssqvtqelra Imdetmkelk aykseleeql tpvaeetrar Iskelqaaqa rlgadmedvc grlvqyrgev qamlgqstee Irvrlashlr klrkrllrda ddlqkrlavy qagaregaer glsairerlg plveqgrvra atvgslagqp Iqeraqawge rlrarmeemg srtrdrldev keqvaevrak leeqaqqirl qaeafqarlk swfeplvedm qrqwaglvek vqaavgtsaa pvpsdnh.
**SEQ ID NO 2** Protein corresponding to chain A of human antithrombin lii complex (Accession number in GenBank: GI 999513 1ATH_A; 432 amino acids): hgspvdicta kprdipmnpm ciyrspekka tedegseqki peatnrrvwe Iskansrfat tfyqhladsk ndndniflsp Isistafamt klgacndtlq qlmevfkfdt isektsdqih fffaklncrl yrkanksskl vsanrlfgdk sltfnetyqd iselvygakl qpldfkenae qsraainkwv snktegritd vipseainel tvlvlvntiy fkglwkskfs pentrkelfy kadgescsas mmyqegkfry rrvaegtqvl elpfkgddit mvlilpkpek slakvekelt pevlqewlde leemmlvvhm prfriedgfs Ikeqlqdmgl vdlfspeksk Ipgivaegrd dlyvsdafhk aflevneegs eaaastavvi agrslnpnrv tfkanrpflv firevplnti ifmgrvanpc vk.
**SEQ ID NO 3** Protein corresponding to chain A of human serum albumin (Accession number in GenBank: GI 122920512 2I2Z_A; 585 amino acids):
   dahksevahr fkdlgeenfk alvliafaqy lqqcpfedhv klvnevtefa ktcvadesae ncdkslhtlf gdklctvatl retygemadc cakqeperne cflqhkddnp nlprlvrpev dvmctafhdn eetflkkyly eiarrhpyfy apellffakr ykaafteccq aadkaacllp kldelrdegk assakqrlxc aslqkfgera fkawavarls qrfpkaefae vsklvtdltk vhtecchgdl lecaddradl akyicenqds issklkecce kpllekshci aevendempa dlpslaadfv eskdvcknya eakdvflgmf lyeyarrhpd ysvvlllrla ktyettlekc caaadphecy akvfdefkpl veepqnlikq ncelfeqlge ykfqnallvr ytkkvpqvst ptlvevsrnl gkvgskcckh peakrmpcae dylsvvlnql cvlhektpvs drvtkcctes Ivnrrpcfsa levdetyvpk efnaetftfh adictlseke rqikkqtalv elvkhkpkat keqlkavmdd faafvekcck addketcfae egkklvaasq aalgl.
**SEQ ID NO 4** Protein corresponding to complement C3 precursor [Homo sapiens]; Accession number in GenBank: GI 115298678 NP_000055; 1663 amino acids):
   mgptsgpsll llllthlpla Igspmysiit pnilrlesee tmvleahdaq gdvpvtvtvh dfpgkklvls sektvltpat nhmgnvtfti panrefksek grnkfvtvqa tfgtqvvekv vlvslqsgyl fiqtdktiyt pgstvlyrif tvnhkllpvg rtvmvnienp egipvkqdsl ssqnqlgvlp Iswdipelvn mgqwkirayy enspqqvfst efevkeyvlp sfevivepte kfyyiynekg levtitarfl ygkkvegtaf vifgiqdgeq rislpeslkr ipiedgsgev vlsrkvlldg vqnpraedlv gkslyvsatv ilhsgsdmvq aersgipivt spyqihftkt pkyfkpgmpf dlmvfvtnpd gspayrvpva vqgedtvqsl tqgdgvakls inthpsqkpl sitvrtkkqe Iseaeqatrt mqalpystvg nsnnylhlsv Irtelrpget Invnfllrmd raheakiryy tylimnkgrl Ikagrqvrep gqdlvvlpls ittdfipsfr Ivayytliga sgqrevvads vwvdvkdscv gslvvksgqs edrqpvpgqq mtlkiegdhg arvvlvavdk gvfvlnkknk ltqskiwdvv ekadigctpg sgkdyagvfs dagltftsss gqqtaqrael qcpqpaarrr rsvqltekrm dkvgkypkel rkccedgmre npmrfscqrr trfislgeac kkvfldccny itelrrqhar ashlglarsn Idediiaeen ivsrsefpes wlwnvedlke ppkngistkl mniflkdsit tweilavsms dkkgicvadp fevtvmqdff idlrlpysvv rneqveirav lynyrqnqel kvrvellhnp afcslattkr rhqqtvtipp ksslsvpyvi vplktglqev evkaavyhhf isdgvrkslk vvpegirmnk tvavrtldpe rlgregvqke dippadlsdq vpdtesetri llqgtpvaqm tedavdaerl khlivtpsgc geqnmigmtp tviavhylde teqwekfgle krqgalelik kgytqqlafr qpssafaafv krapstwlta yvvkvfslav nliaidsqvl cgavkwlile kqkpdgvfqe dapvihqemi gglrnnnekd maltafvlis lqeakdicee qvnslpgsit kagdfleany mnlqrsytva iagyalaqmg rlkgpllnkf Ittakdknrw edpgkqlynv eatsyallal lqlkdfdfvp pvvrwlneqr yygggygstq atfmvfqala qyqkdapdhq elnldvslql psrsskithr ihwesasllr seetkenegf tvtaegkgqg tlsvvtmyha kakdqltcnk fdlkvtikpa petekrpqda kntmileict ryrgdqdatm sildismmtg fapdtddlkq langvdryis kyeldkafsd rntliiyldk vshseddcla fkvhqyfnve liqpgavkvy ayynleesct rfyhpekedg klnklcrdel crcaeencfi qksddkvtle erldkacepg vdyvyktrlv kvqlsndfde yimaieqtik sgsdevqvgq qrtfispikc realkleekk hylmwglssd fwgekpnlsy iigkdtwveh wpeedecqde enqkqcqdlg aftesmvvfg cpn.
**SEQ ID NO 5** Protein isolated from Homo sapiens (Accession number in GenBank: GI 194380608 BAG58457; 763 amino acids):
   mrllwgliwa ssfftlslqk prlllfspsv vhlgvplsvg vqlqdvprgq vvkgsvflrn psrnnvpcsp kvdftlsser dfallslqvp lkdakscglh qllrgpevql vahspwlkds Isrttniqgi nllfssrrgh lflqtdqpiy npgqrvryrv faldqkmrps tdtitvmven shglrvrkke vympssifqd dfvipdisep gtwkisarfs dglesnsstq fevkkyvlpn fevkitpgkp yiltvpghld emqldiqary iygkpvqgva yvrfgllded gkktffrgle sqtklvngqs hislskaefq daleklnmgi tdlqglrlyv aaaiiespgg emeeaeltsw yfvsspfsld Isktkrhlvp gapfllqalv remsgspasg ipvkvsatvs spgsvpevqd iqqntdgsgq vsipiiipqt iselqlsvsa gsphpaiarl tvaappsggp gflsierpds rpprvgdtln Inlravgsga tfshyyymil srgqivfmnr epkrtltsvs vfvdhhlaps fyfvafyyhg dhpvanslrv dvqagacegk lelsvdgakq yrngesvklh letdslalva Igaldtalya agskshkpln mgkvfeamns ydlgcgpggg dsalqvfqaa glafsdgdqw tlsrkrlscp kekttrkkrn vnfqkainek lgqyasptak rccqdgvtrl pmmrsceqra arvqqpdcre pflsccqfae slrkksrdkg qeglgspsar spa.
**SEQ ID NO 6** Protein corresponding to Zn-alpha-2-glycoprotein isolated from Homo sapiens (Accession number in GenBank: GI 38026 CAA42438; 302 amino acids):
   mwasmsrmlp vllslllllg pavpqenqdg rysltyiytg Iskhvedvpa fqalgslndl qffrynskdr ksqpmglwrq vegmedwkqd sqlqkaredi fmetlkdive yyndsngshv lqgrfgceie nnrssgafwk yyydgkdyie fnkeipawvp fdpaaqitkq kweaepvyvq rakayleeec patlrkylky sknildrqdp psvvvtshqa pgekkklkcl aydfypgkid vhwtragevq epelrgdvlh ngngtyqswv vvavppqdta pyschvqhss laqplvvpwe as.

### DETAILED DESCRIPTION OF THE INVENTION

The present invention provides an *in vitro* method for the diagnosis of endometriosis in a subject.

Object of the method described herein is to allow the preferably early diagnosis of endometriosis in female subjects in the absence or in the presence even only of slight symptoms associable to the disease. In particular, it is possible to carry out the diagnosis not only in each one of the conventional four stages of disease, but also before its clinical representation.
In particular, the method is characterized by the determining of the concentration of at least one protein comprising or consisting in an amino acid sequence selected from the group of: SEQ ID NO 1, SEQ ID NO 2, SEQ ID NO 3, SEQ ID NO 4, SEQ ID NO 5 and SEQ ID NO 6 in a biological sample of a subject under examination.
In a preferred embodiment of the present invention, said protein comprises or consists in SEQ ID NO 6. In fact, as mentioned in the foregoing, the Authors of the present invention demonstrated that the variation of the concentration of at least one of the above proteins correlates only with the presence or absence of endometriosis. Specifically, said at least one protein of which the concentration has to be determined is a protein comprising inside its amino acid sequence either SEQ ID NO 1 or SEQ ID NO 2 or SEQ ID NO 3 or SEQ ID NO 4 or SEQ ID NO 5 or SEQ ID NO 6. In other terms, therefore, according to the method described herein, the detectable and/or detected proteins are the proteins (schematically denoted as proteins A-F) below:
- protein A comprising or consisting in SEQ ID NO 1,
- protein B comprising or consisting in SEQ ID NO 2,
- protein C comprising or consisting in SEQ ID NO 3,
- protein D comprising or consisting in SEQ ID NO 4,
- protein E comprising or consisting in SEQ ID NO 5 and/or
- protein F comprising or consisting in SEQ ID NO 6.

In particular, in a preferred embodiment of the invention the detectable and/or detected proteins have an amino acid sequence consisting exclusively in: SEQ ID NO 1 or SEQ ID NO 2 or SEQ ID NO 3 or SEQ ID NO 4 or SEQ ID NO 5 or SEQ ID NO 6.

The detectable and/or detected proteins to the ends of the present invention may be one or two, three, four, five or six and according to any one of the possible combinations. Merely by way of example, when the proteins to be detected (detectable) and/or detected are three, these may be protein A (SEQ ID NO 1), protein D (SEQ ID NO 4) and protein F (SEQ ID NO 5) or protein B (SEQ ID NO 2), protein C (SEQ ID NO 3) and protein F (SEQ ID NO 6), or again protein A (SEQ ID NO 1), protein C (SEQ ID NO 3) and protein F (SEQ ID NO 6). Apparently, any possible combination of the 6 proteins defined above may be utilized to the ends of the present method and, therefore, is as such comprised within the protective scope of the invention described herein.
Moreover, comprised within the protective scope defined herein are also mutated sequences and/or post-translational variants of the sequences defined by SEQ ID NO 1-6.

"Mutated sequences" in the present invention signifies an amino acid sequence X having, with respect to the amino acid sequence indicated in SEQ ID NO 1 or SEQ ID NO 2 or SEQ ID NO 3 or SEQ ID NO 4 or SEQ ID NO 5 or SEQ ID NO 6, at least 90% homology. In other terms, at least the 90, 91, 92, 93, 94, 95, 96, 97, 98, 99 % of the amino acid sequence could be identical to each of the above-defined SEQ IDs.

"Post-translational variants" instead signifies amino acid sequences differing from the sequences SEQ ID NO 1-6, or mutants thereof, for the presence of one or more amino acids on which functional groups like, by way of a non-limiting example, glucidic groups, phosphate, acetyl have been added.

Accordingly, in the carrying out of the method described herein, proteins comprising or consisting in mutated sequences and/or post-translational variants of SEQ ID NO 1-6 may also be detected.

The determining of the concentration of the above proteins can be performed according to any one of the methods deemed suitable therefor by the technician in the field. Such methods are widely known in the literature and described in detail in most laboratory manuals, therefore it is not necessary to further delve into them herein.

Merely by way of example, the concentration of the above proteins in a sample, with respect to a control sample, can be determined by quantitative and semiquantitative immunometric methods. In particular, such methods are based on the recognizing by specific antibodies of the sequences represented by SEQ ID NO 1-6.

The development of an antibody capable of selectively recognizing a given amino acid sequence by now falls within conventional laboratory techniques; in fact, said development is not only described in laboratory manuals, but also performed, upon request, as a service by several biotechnology companies. Therefore, today it is possible to develop an antibody, both of polyclonal and monoclonal type, e.g., against SEQ ID NO 1-6, simply by providing to a company, like e.g. Antibody Resource (http://www.antibodyresource.com/customantibody.html) the sequences of interest in order to obtain a specific antibody for each of SEQ ID NO 1-6.

Preferably, the antibodies developed according to the present invention are monoclonal antibodies.

For completeness of information, let us recall that to make the monoclonal-type primary antibody any standard technique for developing monoclonal antibodies will suffice, like e.g. that defined by Koler and Mirstaein in 1967: It should be briefly recalled herein that each specific antibody, recognizing a specific antigenic determinant (epitope), is produced by a specific B lymphocyte. Isolation and *in vitro* culture of a cell able to produce a single antibody represents a source of monoclonal antibodies (therefore, monospecific antibodies). However, B lymphocytes, when cultivated *in vitro,* die after a very short time and therefore cannot be a source for long-term production of antibodies.
Monoclonal antibody technology comprises the isolation of these B lymphocytes, and their subsequent fusion with transformed cells (myelomatous cells), useful for their features of greater growth and survival. Many of the resulting hybrid cells (or hybridomas), which are cultivated *in vitro,* will retain immortality, besides producing large amounts of the monoclonal antibody.
Fusion between B lymphocytes (coming from the spleen and lymph nodes of an immunized animal) and mouse myeloma (the animal most used), is obtained by intervention of a membrane fusion promoter, like polyethylene glycol.
The medium on which hybrids are grown is of selective type, known under the name of HAT, that, just owing to its composition, inhibits the growth both of myelomas and non-fused spleen cells, but not of the hybridoma completing the two parent lines. Hybridomas are subjected to screening for searching specific antibodies of interest, and those selected are forwarded to storage or mass production.

Antibodies, both polyclonal and monoclonal ones, specific for the above-defined amino acid sequences, are therefore (primary) antibodies which specifically recognize each of the above sequences, and which in turn can then be recognized by a suitable secondary antibody, of course specific for the organism in which the primary antibody has been developed. Such secondary antibody could be labeled, for instance, with any fluorochrome commonly used in secondary antibody labeling, like, e.g., fluorescent substances (by way of example: FITC, Cy3, Cy5, Alexa 488, PEe) or enzymes or substances detectable by enzyme cytochemistry (e.g., radish peroxidase) to thereby allow detecting of the primary antibody and, therefore, of the protein(s) of interest according to conventional detection methods.

Preferably, therefore, the determining of the concentration of at least one of the above-indicated proteins can be performed, by way of example, by western-blot, ELISA (enzyme-linked immunosorbent assay), RIA (radioimmunoassay), immunochemistry or protein arrays.

In particular, a protein array consists of a solid support on which various reagents, among which, e.g., antibodies specific for the proteins described herein, are deposited ("spotted", in technical jargon) in an orderly manner and at a specific and defined density. Each of these antibodies, by binding its own target protein and thereby isolating it from a complex mixture, such as may be, e.g., a cell lysate, allows, on the basis of protein(antigen)-protein (antibody) interactions occurred, to highlight and quantify the specific protein of interest.

Alternatively, the determining of the concentration of at least one of the proteins described herein can be performed by mass spectrometry.
Next, the value related to the concentration of said at least one protein of interest present in a biological sample of the subject under examination is compared with the control value, i.e. the concentration value obtained for the same protein in a sample belonging to a healthy subject. As it will be apparent to a technician in the field, the control value will preferably be the average value of the concentration of said at least one protein calculated with respect to a cohort of healthy subjects.

According to the method for the diagnosis and/or the evaluation of the risk of developing endometriosis described herein, the variation of the concentration of said at least one protein comprising a sequence selected from the group of: SEQ ID NO 1, SEQ ID NO 2, SEQ ID NO 3, SEQ ID NO 4, SEQ ID NO 5 and SEQ ID NO 6 or mutants and/or post-translational variants thereof in the sample of the subject under examination with respect to the control value will provide information about the presence of endometriosis or the risk of developing endometriosis .

In particular, if at least one protein comprising one among SEQ ID NO 1, SEQ ID NO 4, SEQ ID NO 5 proves to have a statistically lower concentration with respect to the same protein analyzed in the control sample, endometriosis or risk of developing endometriosis will be diagnosed to the subject under examination.

Moreover, if at least one protein comprising one among SEQ ID NO 2, SEQ ID NO 3, SEQ ID 6 proves to have a statistically higher concentration with respect to the same protein in the control sample, endometriosis or risk of developing endometriosis will be diagnosed to the subject under examination.

In a preferred embodiment of the present method, said protein comprises or consists in SEQ ID NO 6.

Therefore, in short, according to the method described herein a decrease of the concentration of said at least one protein comprising one among SEQ ID NO 1, SEQ ID NO 4, SEQ ID NO 5 and/or an increase of the concentration of said protein comprising SEQ ID NO 2, SEQ ID NO 3, SEQ ID NO 6 with respect to said control is indicative of endometriosis or of the risk of developing endometriosis. In particular, by way of example, even the mere observation that only one or two, three among SEQ ID NO 2, SEQ ID NO 3, SEQ ID NO 6 are on the increase, and/or only one or two, three, among SEQ ID NO 1, SEQ ID NO 4, SEQ ID NO 5 are on the decrease with respect to the control, is indicative of the presence of endometriosis.

In order to facilitate the determining of the concentration, preferably, the method described herein can also comprise a preliminary step wherein it is obtained a protein extract from the biological sample to be analyzed.

As highlighted above, the further technical problem solved by the present invention is the development of a noninvasive method for the diagnosis of endometriosis. The Authors have observed a modulation of the expression of the above proteins in serum samples of patients with endometriosis. Therefore, in a preferred embodiment of the invention, the biological sample is represented by a sample of blood or serum of the patient under examination.

Moreover, as evident from the type of pathology at issue, i.e. endometriosis, the patient under examination is a female subject, preferably a human subject. However, any animal in which it is possible to observe endometriosis analogously to a human being, like e.g. in horses, may be considered the "subject" according to what described herein.

A further object of the present description is an *in vitro* method for the monitoring of endometriosis in a subject. The term "monitoring" herein signifies the control of the pattern of the pathological state of a patient in time. Therefore, "monitoring" means serial controls in time of the quantitative variations of the proteins in a subject with respect to the quantitative values of the same protein(s) in the controls. Preferably, such monitoring may be, without being limited thereto, a monitoring of a therapy against endometriosis. Therefore, object of this case is to evaluate before, during and/or after a generic time interval or a therapeutic pathway, a possible improvement or worsening of the pathological state that, in the specific case in which the patient be subjected to therapy, corresponds to the possible advantage or disadvantage of the prescribed therapy to treat and/or slow down and/or prevent endometriosis.

In particular, said monitoring method comprises the key step of determining the concentration of at least one protein comprising a sequence selected from the group of: SEQ ID NO 1, SEQ ID NO 2, SEQ ID NO 3, SEQ ID NO 4, SEQ ID NO 5 and SEQ ID NO 6. In a preferred embodiment of the present invention said protein comprises or consists in SEQ ID NO 6. Analogously to what already mentioned for the above diagnosis method, the determining of the concentration of said at least one protein may be related both to proteins comprising and consisting exclusively in SEQ ID NO 1-6, as well as to mutants and/or post-translational variants thereof. In particular, the technical aspects related to the proteins to be analyzed, as well as the methodologies for determining protein concentration, useful to the ends of the monitoring method, are to be considered analogous to those already described above for the diagnosis method. Therefore, in particular, the method could further comprise a step of obtaining a protein extract from said biological samples which, preferably, are samples of blood or serum.

The determining of the protein concentration to the ends of the monitoring should be performed in a first biological sample and in at least one second biological sample of a subject, obtained respectively at a time t=0 and t>0. The subject under examination may be, in particular, both a subject undergoing therapy against endometriosis and a subject monitored in time without necessarily undergo any type of therapy. In other terms, therefore, the first biological sample obtained at time t=0 may be a sample acquired, in a subject undergoing therapy against endometriosis, e.g., before the start of the therapy itself, and instead in a subject not undergoing therapy, acquired at a generic time t=0a. Otherwise, the second biological sample may be acquired at one or more time intervals from said time t=0, therefore defined as times t>0, e.g., every 15, 20, 30 days. Preferably, the first and second sample are respectively obtained prior to initiation of a therapy against endometriosis and during and/or after said therapy. Then, a comparison between the concentration obtained in said first and said at least second sample for a same protein, among those indicated herein, will provide information about the course of the patient's pathological state.

The variation of the concentration of at least one of the proteins comprising or consisting in SEQ ID NO 1-6 between the two samples analyzed is, therefore, the instrument allowing to the clinician an evaluation of the effectiveness or non-effectiveness of the selected therapeutic strategy. In particular, a decrease of the concentration of said at least one protein comprising or consisting in SEQ ID NO 1, SEQ ID NO 4 or SEQ ID NO 5 and/or an increase of the concentration of said protein comprising or consisting in SEQ ID NO 2, SEQ ID NO 3 or SEQ ID NO 6 in said first sample with respect to said at least second one is indicative of a progression of endometriosis, in other terms therefore of a scarcely effective therapy. Vice versa, an increase of the concentration of said at least one protein comprising or consisting in SEQ ID NO 1, SEQ ID NO 4 or SEQ ID NO 5 and/or a decrease of the concentration of said protein comprising or consisting in SEQ ID NO 2, SEQ ID NO 3 or SEQ ID NO 6 in said first sample with respect to said at least second one is indicative of the effectiveness of the therapy against endometriosis. Alternatively, a non-variation of the concentration of the above proteins in said samples might indicate a slowing down and/or stopping of the progression of endometriosis in the patient.

As already highlighted, the type of therapy to be monitored is a generic therapy against endometriosis. In particular, the therapy may also be a surgical-type treatment.

Object of the present description is also the use of a kit for the diagnosis of endometriosis and/or the monitoring of endometriosis in a subject. Preferably, the kit may be a kit for the monitoring of a therapy against endometriosis. In particular, said kit comprises at least one aliquot of one or more reagents necessary to the determining of the concentration of at least one protein comprising or consisting in a sequence selected from the group of: SEQ ID NO 1, SEQ ID NO 2, SEQ ID NO 3, SEQ ID NO 4, SEQ ID NO 5 and SEQ ID NO 6, or mutants and/or post-translational variants thereof, in a biological sample of said subject. In a preferred embodiment of the present invention, said one or more reagents are necessary to the determining of the concentration of a protein comprising or consisting in SEQ ID NO 6.

Preferably, the kit according to the invention could contain one or more aliquots of at least one specific primary monoclonal or polyclonal antibody against one of the sequences defined in SEQ ID NO. 1, SEQ ID NO. 2, SEQ ID NO. 3, SEQ ID NO. 4, SEQ ID NO. 5 or SEQ ID NO. 6. The kit could also contain one or more aliquots of a labeled or unlabeled secondary antibody, said secondary antibody being of course specific for the immune system from which the primary antibody was made. Therefore, if the primary antibody is made in mouse, the secondary one will be anti-mouse, if made in mouse will be anti-rabbit, etc.

The kit could further contain negative controls and/or positive controls. In particular, the kit can contain as positive control one or more aliquots comprising one or more amino acid sequences selected from the group of: SEQ ID NO 1, SEQ ID NO 2, SEQ ID NO 3, SEQ ID NO 4, SEQ ID NO 5 and SEQ ID NO 6.
Moreover, the kit may comprise suitable reagents and means for the procedure of detecting the above proteins by use of antibodies, such as the PBS buffer or other reagents commonly used for antibody detection.
The following examples and experimental results have the purpose of indicating ways of embodiment of the present description, without however being limitative thereof.

### EXAMPLES

### Example 1: Biological material collection and storage

Ten plasma samples, respectively from 5 patients with ascertained endometriosis, after serum collection with surgery and histological examination, and from 5 healthy control patients, were collected and stored at the temperature of -80°C. In detail, blood collections performed by peripheral vein pricking were centrifuged at 3.000 rpm for 10 minutes at 4°C, and the plasma obtained aliquoted and stored at - 80°C.

### Example 2: Proteomics analysis by two-dimensional gel method

The plasma samples were depleted in advance of the most abundant proteins present in the serum (albumin, IgG, antitrypsin, IgA, transferrin and aptoglobine), so as to be able to highlight with greater clarity proteins differently expressed in the two groups of patients. Then, proteins were extracted from 10 plasma samples and said proteins were separated by electrophoresis on two-dimensional gels (20x30 cm 2DE gel). The proteins were highlighted on the gel by a silver-based stain, suitable to a subsequent Mass Spectrometry application. By use of dedicated software, images of the various gels were compared to single out spots expressed in a constantly and significantly different manner in the two groups of patients. Appropriate statistic tests were used to confirm the statistic validity of these differences. This analysis singled out 5 spots significantly and constantly expressed in a different manner in patients with endometriosis with respect to healthy control patients.
By a mass spectrometry method (LC ESI MS/MS), the proteins corresponding to such differentially expressed spots were singled out. Identification of the proteins corresponding to the spots identified by 2D gel was performed by nanoLC-ESI-MS/MS technology. The MS apparatus was a system of an Agilent 1100 nanoLC system (Agilent, Waldbronn, Germany), a NanoMate 100 (Advion, Ithaca, USA) and a Finnigan LTQ-FT mass spectrophotometer (ThermoFisher, Bremen, Germany). Protein spots were digested directly in-gel with trypsin (Promega, Mannheim, Germany) and applied to the nanoLC-ESI-MS/MS system. Peptides produced by protein spot digestion were trapped on a specific enrichment column (Zorbax SB C18, 0.3 x 5 mm, Agilent) for five minutes, using 1% acetonitrile/0.5% formic acid as eluent; then, the peptides were separated on a Zorbax 300 SB C18 column, 75 µm x 150 mm (Agilent) by using a gradient of 0.1% acetonitrile/formic acid from 5% to 40% of acetonitrile for a 40-min period. Mass spectra were automatically recorded by mass spectrometer, following the use conditions indicated by the manufacturer for nanoLC-ESI-MSMS analyses. The corresponding proteins were then identified by using the MS/MS research system of Mascot search engine (Matrix Science, London, England) and the appropriate protein database (National Center for Biotechnology Information, Bethesda, USA).

The proteins singled out through said method are respectively:
**1)** Protein corresponding to human apolipoprotein E mutant E3K (SEQ ID NO:1);
**2)** Protein corresponding to chain A of human antithrombin lii complex (SEQ ID NO:2);
**3)** Protein corresponding to chain A of human serum albumin (SEQ ID NO:3);
**4)** Protein corresponding to precursor C3 of the complement (SEQ ID NO:4);
**5)** Protein isolated from Homo sapiens (SEQ ID NO:5);
**6)** Protein corresponding to glycoprotein Zn-alpha 2 (SEQ ID NO:6)

In Figures 1A-F are shown the histograms showing the different expression of the above proteins in a serum sample of patients with endometriosis, with respect to serum samples of a control population.

### REFERENCE

- Fassbender A, Waelkens E, Verbeeck N, Kyama CM, Bokor A, Vodolazkaia A, Van de Plas R, Meuleman C, Peeraer K, Tomassetti C, Gevaert O, Ojeda F, De Moor B, D'Hooghe T: Proteomics analysis of plasma for early diagnosis of endometriosis. Obstet Gynecol 2012, 119: 276-285.
- Giudice LC , and Kao LC: Endometriosis. The Lancet, 364: 1789-1799, 2004.
- Hadfield R, Mardon H, Barlow D, Kennedy S. Delay in the diagnosis of endometriosis: a survey of women from the USA and the UK. Hum Reprod 1996, 11: 878-880.
- Houston DE: Evidence for the risk of pelvic endometriosis by age, race, and socioeconomic status. Epidemiol Rev, 6: 167-191, 1984.
- May KE, Conduit-Hulbert SA, Villar J, Kirtkley S, Kennedy SH, Becker CM: Peripheral biomarkers of endometriosis: a systematic review. Hum Reprod 2010, 16: 651-674.

## Claims

1. An *in vitro* method for the diagnosis of endometriosis in a subject, comprising the following steps:
a) determining the concentration of SEQ ID NO 6, (Zn-alpha-2-glycoprotein (ZAG)) or mutants and/or post-translational variants thereof, in a biological sample of said subject and in a control sample;
b) comparing said concentration in said biological sample and said control sample
wherein an increase of the concentration of said SEQ ID NO 6 with respect to the same protein in said control is indicative of endometriosis.

2. The method according to claim 1, wherein said step a) further comprises the determining of at least one protein comprising a sequence selected from the group of: SEQ ID NO 5, SEQ ID NO 4, SEQ ID NO 3, SEQ ID NO 2, SEQ ID NO 1, or mutants and/or post-translational variants thereof in a biological sample of said subject.
wherein an increase of the concentration of said at least SEQ ID NO 3, SEQ ID NO 2 and/or a decrease of the concentration of said at least one protein comprising a sequence selected from the group of SEQ ID NO 5, SEQ ID NO 4, SEQ ID NO 1 with respect to the same protein in said control is indicative of endometriosis.

3. The method according to claim 1 or 2, further comprising a step of obtaining a protein extract from said biological sample.

4. The method according to any one of the claims 1-3, wherein said biological sample is blood or serum.

5. The method according to any one of the claims 1-4, wherein said determining is performed by western-blot, ELISA (enzyme-linked immunosorbent assay), RIA (radioimmunoassay), immunochemistry, protein arrays, mass spectrometry.

6. An *in vitro* method for the monitoring of endometriosis in a subject, comprising the following steps:
a) determining the concentration of at least one protein comprising SEQ ID NO 6, or mutants and/or post-translational variants thereof, in a first and in a second biological sample, obtained respectively at a time t=0 and t>0, of said subject,
b) comparing said concentration obtained for said first and second sample.

7. The method according to claim 6, wherein said step a) further comprises the determining of at least one protein comprising a sequence selected from the group of: SEQ ID NO 5, SEQ ID NO 4, SEQ ID NO 3, SEQ ID NO 2, SEQ ID NO 1, or mutants and/or post-translational variants thereof, in a biological sample of said subject.

8. The method according to claim 7, wherein an increase of the concentration of said protein comprising a sequence selected from the group of SEQ ID NO 6, SEQ ID NO 3, SEQ ID NO 2 and/or a decrease of the concentration of said at least one protein comprising a sequence selected from the group of SEQ ID NO 5, SEQ ID NO 4, SEQ ID NO 1 in said first sample with respect to said second sample is indicative of a progression of endometriosis.

9. The method according to any one of the claims 6 to 8, wherein said first and second sample are respectively obtained prior to initiation of a therapy and during and/or after said therapy.

10. The method according to any one of the claims 6 to 9, further comprising a step of obtaining a protein extract from said biological samples.

11. The method according to any one of the claims 6 to 10, wherein said biological samples are blood or serum.

12. The method according to any one of the claims 9 to 11, wherein said therapy is a surgical treatment.

13. Use of a kit comprising:
- at least one aliquot of one or more reagents necessary to the determining of the concentration of at least one protein comprising SEQ ID NO 6, (Zn-alpha-2-glycoprotein (ZAG)) or mutants and/or post-translational variants thereof, in a biological sample of said subject and at least one aliquot of one or more reagents necessary to the determining of the concentration of at least one protein comprising a sequence selected from the group of: SEQ ID NO 5, SEQ ID NO 4, SEQ ID NO 3, SEQ ID NO 2, SEQ ID NO 1, or mutants and/or post-translational variants thereof, in a biological sample of said subject in a method according to claim 1.

14. Use of the kit according to claim 13, further comprising at least one aliquot of a positive control comprising one or more amino acid sequences selected from the group of: SEQ ID NO 6, SEQ ID NO 5, SEQ ID NO 4, SEQ ID NO 3, SEQ ID NO 2 and SEQ ID NO 1.

## Patentansprüche

1. *In vitro*-Verfahren zur Diagnose von Endometriose bei einem Individuum, umfassend die folgenden Schritte:
a) Bestimmen der Konzentration von SEQ ID NO:6 (Zn-alpha-2-Glycoprotein; ZAG) oder Mutanten und/oder posttranslationalen Varianten davon in einer biologischen Probe des Individuums und in einer Kontrollprobe;
b) Vergleichen der Konzentration in der biologischen Probe und in der Kontrollprobe,
wobei eine Erhöhung der Konzentration der SEQ ID NO:6 in Bezug auf das gleiche Protein in der Kontrolle auf Endometriose hindeutet.

2. Verfahren nach Anspruch 1, wobei der Schritt a) des Weiteren das Bestimmen mindestens eines Proteins umfasst, das eine Sequenz umfasst, die ausgewählt ist aus der Gruppe aus: SEQ ID NO:5, SEQ ID NO:4, SEQ ID NO:3, SEQ ID NO:2, SEQ ID NO:1 oder Mutanten und/oder posttranslationalen Varianten davon, in einer biologischen Probe des Individuums,
wobei eine Erhöhung der Konzentration von mindestens SEQ ID NO:3, SEQ ID NO:2 und/oder eine Verringerung der Konzentration mindestens eines Proteins, das eine Sequenz umfasst, die ausgewählt ist aus der Gruppe aus SEQ ID NO:5, SEQ ID NO:4, SEQ ID NO:1, in Bezug auf das gleiche Protein in der Kontrolle auf Endometriose hindeutet.

3. Verfahren nach Anspruch 1 oder 2, das des Weiteren einen Schritt des Erhaltens eines Proteinextrakts aus der biologischen Probe umfasst.

4. Verfahren nach einem der Ansprüche 1 bis 3, wobei die biologische Probe Blut oder Serum ist.

5. Verfahren nach einem der Ansprüche 1 bis 4, wobei das Bestimmen durch Western-Blot, Enzymimmunassay (enzyme-linked immunosorbent assay; ELISA), Radioimmunassay (RIA), Immunchemie, Protein-Arrays, Massenspektrometrie durchgeführt wird.

6. *In vitro*-Verfahren zur Überwachung von Endometriose bei einem Individuum, umfassend die folgenden Schritte:
a) Bestimmen der Konzentration mindestens eines Proteins, das SEQ ID NO:6 umfasst, oder von Mutanten und/oder posttranslationalen Varianten davon, in einer ersten und einer zweiten biologischen Probe, die jeweils zu einer Zeit t=0 und t>0 erhalten wird, des Individuums,
b) Vergleichen der Konzentration, die für die erste und zweite Probe erhalten wurde.

7. Verfahren nach Anspruch 6, wobei der Schritt b) des Weiteren das Bestimmen mindestens eines Proteins umfasst, das eine Sequenz umfasst, die ausgewählt ist aus der Gruppe aus: SEQ ID NO:5, SEQ ID NO:4, SEQ ID NO:3, SEQ ID NO:2, SEQ ID NO:1 oder Mutanten und/oder posttranslationalen Varianten davon, in einer biologischen Probe des Individuums.

8. Verfahren nach Anspruch 7, wobei eine Erhöhung der Konzentration des Proteins, das eine Sequenz umfasst, die ausgewählt ist aus SEQ ID NO:6, SEQ ID NO:3, SEQ ID NO:2 und/oder eine Verringerung der Konzentration mindestens eines Proteins, das eine Sequenz umfasst, die ausgewählt ist aus der Gruppe aus SEQ ID NO:5, SEQ ID NO:4, SEQ ID NO:1, in der ersten Probe in Bezug auf die zweite Probe auf ein Fortschreiten der Endometriose hindeutet.

9. Verfahren nach einem der Ansprüche 6 bis 8, wobei die erste und die zweite Probe jeweils vor Beginn einer Therapie und während und/oder nach der Therapie erhalten werden.

10. Verfahren nach einem der Ansprüche 6 bis 9, das des Weiteren einen Schritt des Erhaltens eines Proteinextrakts aus den biologischen Proben umfasst.

11. Verfahren nach einem der Ansprüche 6 bis 10, wobei die biologischen Proben Blut oder Serum sind.

12. Verfahren nach einem der Ansprüche 9 bis 11, wobei die Therapie eine chirurgische Behandlung ist.

13. Verwendung eines Kits, umfassend:
mindestens ein Aliquot eines oder mehrerer Reagenzien, die nötig sind für die Bestimmung der Konzentration mindestens eines Proteins, das SEQ ID NO:6 (Zn-alpha-2-Glycoprotein; ZAG) umfasst, oder Mutanten und/oder posttranslationalen Varianten davon, in einer biologischen Probe des Individuums und mindestens ein Aliquot eines oder mehrerer Reagenzien, die nötig sind für das Bestimmen der Konzentration mindestens eines Proteins, das eine Sequenz umfasst, die ausgewählt ist aus der Gruppe aus: SEQ ID NO:5, SEQ ID NO:4, SEQ ID NO:3, SEQ ID NO:2, SEQ ID NO:1, oder Mutanten und/oder posttranslationalen Varianten davon, in einer biologischen Probe des Individuums in einem Verfahren nach Anspruch 1.

14. Verwendung des Kit nach Anspruch 13, des Weiteren umfassend mindestens ein Aliquot einer Positivkontrolle, die eine oder mehrere Aminosäuresequenzen umfasst, die ausgewählt sind aus der Gruppe aus: SEQ ID NO:6, SEQ ID NO:5, SEQ ID NO:4, SEQ ID NO:3, SEQ ID NO:2 und SEQ ID NO:1.

## Revendications

1. Procédé *in vitro* pour le diagnostic de l'endométriose chez un sujet, comprenant les étapes suivantes :
a) la détermination de la concentration de SEQ ID NO : 6 (Zn-alpha-2-glycoprotéine (ZAG)) ou de ses mutants et/ou variants posttraductionnels, dans un échantillon biologique dudit sujet et dans un échantillon témoin ;
b) la comparaison de ladite concentration dans ledit échantillon biologique et ledit échantillon témoin
dans lequel une augmentation de la concentration de ladite SEQ ID NO : 6 par rapport à la même protéine dans ledit témoin indique une endométriose.

2. Procédé selon la revendication 1, dans lequel ladite étape a) comprend en outre la détermination d'au moins une protéine comprenant une séquence choisie dans le groupe de : SEQ ID NO : 5, SEQ ID NO : 4, SEQ ID NO : 3, SEQ ID NO : 2, SEQ ID NO : 1, ou leurs mutants et/ou variants posttraductionnels dans un échantillon biologique dudit sujet,
dans lequel une augmentation de la concentration desdites au moins SEQ ID NO : 3, SEQ ID NO : 2 et/ou une diminution de la concentration de ladite au moins une protéine comprenant une séquence choisie dans le groupe de SEQ ID NO : 5, SEQ ID NO : 4, SEQ ID NO : 1 par rapport à la même protéine dans ledit témoin indique une endométriose.

3. Procédé selon la revendication 1 ou 2, comprenant en outre une étape d'obtention d'un extrait de protéine à partir dudit échantillon biologique.

4. Procédé selon l'une quelconque des revendications 1 à 3, dans lequel ledit échantillon biologique est du sang ou du sérum.

5. Procédé selon l'une quelconque des revendications 1 à 4, dans lequel ladite détermination est effectuée par Western blot, ELISA (enzyme-linked immunosorbent assay), RIA (technique radioimmunologique), immunochimie, puces de protéines, spectrométrie de masse.

6. Procédé *in vitro* pour la surveillance de l'endométriose chez un sujet, comprenant les étapes suivantes :
a) la détermination de la concentration d'au moins une protéine comprenant SEQ ID NO : 6, ou de ses mutants et/ou variants posttraductionnels, dans un premier et dans un second échantillon biologique, obtenus respectivement à un temps t = 0 et t > 0, dudit sujet,
b) la comparaison de ladite concentration obtenue pour lesdits premier et second échantillons.

7. Procédé selon la revendication 6, dans lequel ladite étape a) comprend en outre la détermination d'au moins une protéine comprenant une séquence choisie dans le groupe de : SEQ ID NO : 5, SEQ ID NO : 4, SEQ ID NO : 3, SEQ ID NO : 2, SEQ ID NO : 1, ou leurs mutants et/ou variants posttraductionnels, dans un échantillon biologique dudit sujet.

8. Procédé selon la revendication 7, dans lequel une augmentation de la concentration de ladite protéine comprenant une séquence choisie dans le groupe de SEQ ID NO : 6, SEQ ID NO : 3, SEQ ID NO : 2 et/ou une diminution de la concentration de ladite au moins une protéine comprenant une séquence choisie dans le groupe de SEQ ID NO : 5, SEQ ID NO : 4, SEQ ID NO : 1 dans ledit premier échantillon par rapport au dit second échantillon indique une progression de l'endométriose.

9. Procédé selon l'une quelconque des revendications 6 à 8, dans lequel lesdits premier et second échantillons sont obtenus respectivement avant l'initiation d'un traitement et durant et/ou après ledit traitement.

10. Procédé selon l'une quelconque des revendications 6 à 9, comprenant en outre une étape d'obtention d'un extrait de protéine à partir desdits échantillons biologiques.

11. Procédé selon l'une quelconque des revendications 6 à 10, dans lequel lesdits échantillons biologiques sont du sang ou du sérum.

12. Procédé selon l'une quelconque des revendications 9 à 11, dans lequel ledit traitement est un traitement chirurgical.

13. Utilisation d'un kit comprenant :
- au moins une aliquote d'un ou de plusieurs réactifs nécessaires à la détermination de la concentration d'au moins une protéine comprenant SEQ ID NO : 6 (Zn-alpha-2-glycoprotéine (ZAG)) ou de ses mutants et/ou variants posttraductionnels, dans un échantillon biologique dudit sujet et au moins une aliquote d'un ou de plusieurs réactifs nécessaires à la détermination de la concentration d'au moins une protéine comprenant une séquence choisie dans le groupe de : SEQ ID NO : 5, SEQ ID NO : 4, SEQ ID NO : 3, SEQ ID NO : 2, SEQ ID NO : 1, ou leurs mutants et/ou variants posttraductionnels, dans un échantillon biologique dudit sujet dans un procédé selon la revendication 1.

14. Utilisation du kit selon la revendication 13, comprenant en outre une aliquote d'un témoin positif comprenant une ou plusieurs séquences d'acides aminés choisies dans le groupe de : SEQ ID NO : 6, SEQ ID NO : 5, SEQ ID NO : 4, SEQ ID NO : 3, SEQ ID NO : 2 et SEQ ID NO : 1.
